# EUROPEAN PATENT APPLICATION

(11) **EP 4 095 225 A1**
(43) Date of publication of application: **30.11.2022**
(21) Application number: 20905500.3
(22) Date of filing: 28.12.2020
(51) Int. Cl.: C12M 1/00, C12M 1/12, C12N 5/00

(54) **CELL CULTURE SUBSTRATE AND MANUFACTURING METHOD THEREOF**

(30) Priority: 27.12.2019 KR 20190177038
(71) Applicant: Amolifescience Co., Ltd., Seoul 06527 (KR)
(72) Inventor: KIM, Hyeong Taek, Seoul 06527 (KR); SEO, Dong Sik, Seoul 06527 (KR); GOO, Hui Gwan, Seoul 06527 (KR)
(74) Representative: Hutter, Anton
(86) International application number: PCT/KR2020/019213
(87) International publication number: WO 2021/133142

(57) **Abstract**

A cell culture substrate is provided. According to one embodiment of the present invention, the cell culture substrate includes a substrate provided with a non-porous surface and a cell culture coating layer that covers at least a portion of the non-porous surface, wherein the cell culture coating layer is implemented such that particles formed of fusion proteins for culturing cells are aggregated to form at least a portion of the surface, the fusion proteins comprising functional peptides bound to mussel adhesive proteins. In particular, despite containing compounds such as proteins that are beneficial to culturing cells, the cell culture substrate can be stored at room temperature for a long period of time over several years, exhibiting excellent storage stability, while activities of such compounds that are beneficial to culturing of the cells remain unchanged or only suffer minimal degradation so that the cells can be cultured at an initially-designed level. Further, the cell culture substrate has an excellent cell adhesion capability that allows reliable proliferation of the cells attached thereto, and thus can achieve a high cell culture efficiency. Accordingly, the cell culture substrate can be widely utilized for culturing various cells including stem cells.

## Description

### [Technical Field]

The present invention relates to a cell culture substrate and manufacturing method thereof.

### [Background Art]

Recently, as the use of cultured cells for disease treatment is expanded, interest and research on cell culture are increasing. Cell culture is a technology for collecting cells from a living body and culturing them outside the living body. The cultured cells can be differentiated into various tissues of the body, such as skin, organs, and nerves, and then transplanted into the human body or the culture cells can be transplanted into the human body in a state before differentiation to achieve engraftment and differentiation at the same time, so that they can be used for treating various diseases.

Cultivation of mammalian cells is one of many processes in the life sciences and health sciences. As a cell culture substrate for mammalian cell culture and analysis including anchoragedependent cells, a vessel such as a well-plate made of, for example, a high molecular polymer or glass, or a plate such as a film, is often used. Here, additional surface treatment is required to allow the cells to adhere to the surface of the vessel or plate. Such surface treatment may include, for example, forming an adsorption layer on the surface or implementing an appropriate surface shape by adsorption, grafting, or plasma polymerization techniques. Alternatively, the surface treatment may be achieved through chemical modification of the surface itself of the container or plate, for example, atmospheric corona, radio frequency vacuum plasma, DC glow discharge, and microwave plasma treatment.

On the other hand, current methods for culturing, differentiating, cross-differentiating, and reprogramming various stem cells including, for example, adult stem cells (ASCs) and pluripotent stem cells and somatic cells, generally require complex culture environments, for example, a microenvironment similar to an extracellular matrix, to culture the stem cells. The microenvironment is formed by forming a coating layer using extracellular matrix proteins or other various proteins helpful for cell proliferation on the surface of a solid substrate.

On the other hand, the coating layer is formed by simply treating a solution containing the above-described various proteins on a cell adhesion surface such as a container or plate and then drying. However, the stability of the protein activity in the coating layer is very low, and there is a problem in that the activity is easily lost within a few hours at room temperature after the coating layer is formed. Therefore, it is difficult to manufacture a cell culture substrate having the coating layer formed in advance, and even if it is manufactured, the cell culture substrate must be stored at a low temperature, and even when stored at a low temperature, the storage days are very short, within 30 days. In addition, due to such poor storage stability, it is common to form the coating layer on the cell adhesion surface immediately before cell loading operation, which causes inconvenience in cell culture operation and prolongs the preparation time before cell culture.

### [DISCLOSURE]

### [Technical Problem]

The present invention has been devised in consideration of the above, and an object of the present invention is to provide a cell culture substrate which can be stored at room temperature for a long period of time over several years, exhibiting excellent storage stability, while activities of compounds that are beneficial to culturing of the cells remain unchanged or only suffer minimal degradation so that the cells can be cultured at an initially-designed level, and a method for manufacturing the same.

Further, another object of the present invention is to provide a cell culture substrate which can have an excellent cell adhesion capability and allow reliable proliferation of the cells attached thereto, and thus can achieve a high cell culture efficiency, and a method for manufacturing the same.

Furthermore, another object of the present invention is to provide a cell culture coating composition that can achieve the above excellent properties.

### [Technical Solution]

In order to achieve the above object, the present invention provides a cell culture substrate, including a substrate having a non-porous surface and a cell culture coating layer covering at least a portion of the non-porous surface, wherein the cell culture coating layer is an aggregation of particles formed of a fusion protein for cell culture in which a functional peptide is bound to a mussel adhesive protein.

According to one embodiment of the present invention, the functional peptide may have a function of promoting any one or more of adhesion, migration, proliferation and differentiation of a cell.

In addition, the substrate may be formed of any one or more materials selected from the group consisting of polycarbonate, polystyrene, polyimide, polyester, polyurethane, and glass.

In addition, the mussel adhesive protein may be any one protein selected from the group consisting of amino acid sequences of SEQ ID NO: 1 to SEQ ID NO: 14, or a protein to which one or more amino acid sequences selected from the group are linked.

In addition, the functional peptide may include an RGD sequence.

In addition, the functional peptide may be any one or more peptides selected from the group consisting of amino acid sequences of SEQ ID NO: 15 to SEQ ID NO: 18, or a peptide to which one or more amino acid sequences selected from the group are linked.

In addition, the present invention provides a method for manufacturing a cell culture substrate, including the steps of (1) preparing an active solution containing a carbodiimide-based coupling agent and a reactive agent and a fusion protein for cell culture in which a functional peptide is bound to a mussel adhesive protein, (2) preparing a cell culture coating composition by mixing the prepared active solution and the prepared fusion protein for cell culture, and (3) forming a cell culture coating layer by treating the cell culture coating composition on a surface of a non-porous substrate.

According to one embodiment of the present invention, the carbodiimide-based coupling agent may be 1-ethyl-3-(3-dimethylaminopropyl carbodiimide hydrochloride (EDC) or N,N'-dicyclohexylcarboimide (DCC), and the reactant may be N-hydroxysuccinimide (NHS) or N-hydroxysulfosuccinimide (Sulfo-NHS), more preferably N-hydroxysulfosuccinimide (Sulfo-NHS).

In addition, the carbodiimide-based coupling agent and the reactive agent may be contained in the active solution in a weight ratio of 1: 0.1 to 10. In the cell culture coating composition, 1 to 100 parts by weight of the carbodiimide-based coupling agent may be mixed with respect to 100 parts by weight of the fusion protein for cell culture.

In addition, the present invention provides a cell culture coating composition for a non-porous cell culture substrate which forms a cell culture coating layer on a surface of the non-porous cell culture substrate, the cell culture coating composition including a fusion protein for cell culture in which a functional peptide is bound to a mussel adhesive protein, a carbodiimide-based coupling agent, and a reactive agent.

Hereinafter, the terms used in the present invention will be described.

The term "extracellular matrix (ECM)" used in the present invention is a matrix that surrounds the outside of a cell, occupies between cells, and means having a network structure mainly composed of proteins and polysaccharides.

### [Advantageous Effect]

Despite containing compounds such as proteins that are beneficial to culturing cells, the cell culture substrate according to the present invention can be stored at room temperature for a long period of time over several years, exhibiting excellent storage stability, while activities of such compounds that are beneficial to culturing of the cells remain unchanged or only suffer minimal degradation so that the cells can be cultured at an initially-designed level. Further, since the cell adhesion capability to the cell culture substrate is excellent, and the cells attached thereto can be reliably proliferated, a high cell culture efficiency can be achieved. Accordingly, the cell culture substrate can be widely utilized for culturing various cells including stem cells.

### [Description of Drawings]

FIGS. 1 and 2 show SEM photographs of surfaces of cell culture substrates according to Examples 1 and 2 of the present invention.
FIG. 3 shows a SEM photograph of a surface of a cell culture substrate according to a comparative example of the present invention.
FIGS. 4 to 7 respectively show a SEM photograph of a surface of a cell culture substrate according to an example of the present invention.
FIGS. 8 and 9 show photographs of results of cell culture through a cell culture substrate according to an example of the present invention and a cell culture substrate according to a comparative example.
FIG. 10 shows photographs of culturing results of four types of cells after applying various types of mediums to a cell culture substrate according to an example of the present invention.
FIGS. 11 to 13 show photographs of culturing results of cells after an accelerated experiment in order to evaluate storage stabilities of a cell culture substrate according to an example of the present invention and a cell culture substrate according to a comparative example.

### [Mode for Invention]

Hereinafter, with reference to the accompanying drawings, the embodiments of the present invention will be described in detail so that those of ordinary skill in the art to which the present invention pertains can easily implement them. The present invention may be embodied in many different forms and is not limited to the embodiments described herein.

A cell culture substrate according to an embodiment of the present invention includes a substrate having a non-porous surface and a cell culture coating layer covering at least a portion of the non-porous surface, wherein the cell culture coating layer includes a function protein for cell culture in which a functional peptide is bound to a mussel adhesive protein.

The substrate is a support for culturing cells, all of which are non-porous, or at least the surface on which the cell culture coating layer to be described later is disposed may be non-porous. In addition, a substrate commonly used in cell culture may be used without limitation for the substrate. As an example, the substrate may be a substrate in the form of a container, commonly called a well plate, or a plate-shaped plate such as a film, but is not limited thereto. In addition, for the material of the substrate, a material of the substrate commonly used in the cell culture may be used without limitation. For example, it may be formed of any one or more materials selected from the group consisting of polycarbonate, polystyrene, polyimide, polyester, polyurethane, and glass.

The surface of the substrate on which the cell culture coating layer is formed may be one that has been subjected to a known surface modification treatment such as plasma treatment, but preferably it may be a surface not subjected to plasma treatment. When the cell culture coating layer to be described later is formed on the plasma-untreated surface of the substrate, it is possible to achieve an increased cell culture efficiency compared to the case where it is formed on the plasma-treated surface of the substrate.

Next, the cell culture coating layer provided on the surface of the above-described substrate will be described.

The cell culture coating layer is a layer that provides a cell adhesion surface on which cells to be cultured are seeded and then settled and proliferated. The cell culture coating layer is formed including a fusion protein for cell culture in which a functional peptide is bound to a mussel adhesive protein. Specifically, the cell culture coating layer that is formed by aggregating the particles formed of the fusion protein for cell culture is provided on the cell adhesion surface. The cell culture coating layer, which is formed through the fusion protein for cell culture and whose surface is implemented in the form of aggregated particles, is excellent in cell culture efficiency. At the same time, even when stored at room temperature for more than several years, storage stability is greatly improved as the decrease in the activity of functional peptides caused by degradation and denaturation of the fusion protein for cell culture forming the cell culture coating layer is prevented or minimized. In addition, the cell culture coating layer does not use a polymer-based adhesive component, for example, an acrylic adhesive component, and introduces a functional peptide to the surface of the cell culture substrate, so there is no cytotoxicity, and the cells can be cultured more biocompatible.

The cell culture coating layer is formed through a cell culture fusion protein in which a functional peptide is bound to a mussel adhesive protein, and the functional peptide is a material having a function to help cell culture. Specifically, it may be a material that performs the function of promoting any one or more of cell adhesion, cell migration, cell proliferation, and cell differentiation. As the functional peptide, known peptides performing these functions may be used without limitation. Non-limiting examples include adrenomedullin, angiopoietin, bone morphogenetic protein (BMP), brain-derived neurotrophic factor (BDNF), epidermal growth factor (EGF), erythropoietin, fibroblast growth factor, glial cell line-derived neurotrophic factor (GDNF), granulocyte colony-stimulating factor (G-CSF), granulocyte macrophage colony-stimulating factor (GM-CSF), growth differentiation factor-9 (GDF9), hepatocyte growth factor (HGF), hepatoma-derived growth factor (HDGF), insulin-like growth factor (IGF), keratinocyte growth factor (KGF), migration-stimulating factor (MSF), myostatin (GDF-8), nerve growth factor (NGF), platelet-derived growth factor (PDGF), thrombopoietin (TPO), T-cell growth factor (TCGF), neuropilin, transforming growth factor-alpha (TGF-α), transforming growth factor-beta (TGF-β), tumor necrosis factor-alpha (TNF-α), vascular endothelial growth factor (VEGF), a predetermined amino acid sequence included in any one or more growth factors (GF) selected from the group consisting of IL-1, IL-2, IL-3, IL-4, IL-5, IL-6 and IL-7. Alternatively, it may include a predetermined amino acid sequence included in any one or one extracellular matrices selected from the group consisting of hyaluronic acid, heparin sulfate, chondroitin sulfate, termatin sulfate, keratan sulfate, alginate, fibrin, fibrinogen, collagen, elastin, fibronectin, vitronectin, cadherin and laminin.

For example, the functional peptide may include an RGD sequence in an amino acid sequence. In addition, the functional peptide may be any one or more peptides selected from the group consisting of the amino acid sequence of SEQ ID NO: 15 to SEQ ID NO: 18 or a peptide to which one or more amino acid sequences selected from the group are linked. In addition, the functional peptide may be a vitronectin polypeptide, a collagen polypeptide, a laminin polypeptide, a fibronectic polypeptide, or a variant thereof.

On the other hand, the functional peptide may be, for example, a peptide having 3 to 100 amino acids, more preferably 3 to 50 amino acids. For example, the functional peptide may be a peptide having 3 to 30 amino acids, and through this, even when stored in a state contained in the coating layer at room temperature for a long time, it may be more advantageous to minimize or prevent degradation, denaturation, and the like.

In addition, the functional peptide is bound to the mussel adhesive protein, and specifically, it may be bound to the carboxy terminus, the amino terminus, or both the carboxy terminus and the amino terminus of the mussel adhesive protein. In this case, the bond may be a covalent bond, specifically, an amino bond. On the other hand, the functional peptide and the mussel adhesive protein can be bound through a known method, and for example, can be prepared through a recombinant protein production method using E. coli. On the other hand, the mussel adhesive protein and the functional peptide may be directly covalently bonded, but the present invention is not limited thereto. It is illustrated that the mussel adhesive protein and functional peptide can be indirectly bound by mediating a predetermined material such as a crosslinking agent.

The reasons for binding the functional peptide to the mussel adhesive protein is that the mussel adhesive protein is advantageous for fixing the functional peptides to the substrate surface with good adhesion properties, and that there is no toxicity that may be applied to cultured cells compared to the polymer-based adhesive component and there is excellent biocompatibility as described above. In addition, there is an advantage in that the dissociation of the seeded cells can be minimized due to good adhesion properties with the seeded cells after the seeded cells are seated on the cell adhesion surface.

The mussel adhesive protein is an adhesive protein derived from mussels, and a known adhesive protein collectively referred to as a mussel adhesive protein may be used without limitation. Preferably, the mussel adhesive protein may be any one protein selected from the group consisting of the amino acid sequences of SEQ ID NO: 1 to SEQ ID NO: 14 or a protein to which one or more amino acid sequences selected from the group are linked. For example, it may be SEQ ID NO: 13.

In addition, it is preferable that the particles are formed in an appropriate amount, and if the aggregated amount is large, that is, if the diameter of the particles becomes large and the number of particles decreases, cell adhesion and cell proliferation may be reduced.

The cell culture substrate in which the above-described cell culture coating layer is provided on the substrate surface according to an embodiment of the present invention may be manufactured by the steps of (1) preparing an active solution containing a carbodiimide-based coupling agent and a reactive agent and the fusion protein for cell culture in which the functional peptide is bound to the mussel adhesive protein, (2) mixing the prepared active solution with the fusion protein for cell culture to prepare a cell culture coating composition, and (3) treating the cell culture coating composition on the surface of a non-porous substrate to form the cell culture coating layer.

First, as the step (1) according to the present invention, the step of preparing the active solution containing a carbodiimide-based coupling agent and a reactive agent and the fusion protein for cell culture in which the functional peptide is bound to the mussel adhesive protein is performed.

The active solution includes a carbodiimide-based coupling agent and a reactive agent, and may further include a solvent. The active solution is a material that introduces the fusion protein for cell culture to the surface of the substrate, and improves the adhesion between the cell culture coating layer and the surface of the substrate compared to the case where the fusion protein for cell culture is simply treated on the surface of the substrate by a conventional method. In addition, the fusion proteins for cell culture are aggregated in a granular shape, there is little risk of infection by external bacteria, stable long-term storage is possible even with temperature changes, and there is little variation in cell culture. On the other hand, it is difficult to say that the granular form in which the aggregation of the fusion proteins is induced by the active solution is due to a specific chemical bond between the fusion proteins, for example, an amino bond between a carboxy group and an amine group by a conventionally known carbodiimide-based coupling agent. This is because a number of hydroxyl groups included in the mussel adhesive protein can also be reacted with the carbodiimide-based coupling agent. Therefore, it is difficult to see that the granular form formed by the fusion protein having multiple reaction sites according to the present invention is due to a specific reaction and the resulting chemical bond, but it can be seen as a unique result that occurs depending on the combination between the active solution and the fusion protein according to the present invention.

For the carbodiimide-based coupling agent, a coupling agent that allows the fusion proteins to bind to each other can be used without limitation. For example, it may be 1-[3-(dimethylamino)propyl]-3-ethylcarboimide hydrochloride (EDC) or N,N'-dicyclohexylcarboimide (DCC).

In addition, the reactive agent is provided to prevent the fusion protein in a coupled state with the carbodiimide-based coupling agent from being hydrated, thereby increasing the efficiency of binding the fusion proteins to each other. For example, it may be N-hydroxysulfosuccinimide (Sulfo-NHS). On the other hand, N-hydroxysuccinimide (NHS), which is conventionally known as a reactive agent, may not be suitable to achieve the desired effect of the present invention.

The active solution may contain the carbodiimide-based coupling agent and the reactive agent in a weight ratio of 1:0.1 to 10. If they are not contained in an appropriate ratio, it is difficult to achieve the desired effect of the present invention, and there is a risk that the cell adhesion in the realized cell culture coating layer is significantly reduced.

In addition, the active solution may further contain sodium acetate to improve reactivity. In this case, the sodium acetate may be contained in an amount of 1 to 100 parts by weight based on 100 parts by weight of the carbodiimide-based coupling agent.

In addition, the active solution may further contain a solvent, and the solvent may be water or an organic solvent, for example water.

The method for preparing the active solution is not particularly limited, but, for example, the final active solution may be prepared by adding sodium acetate solution to the carbodiimide-based coupling agent solution and the reactive agent solution, respectively, and mixing them to prepare two types of solutions, and then, mixing the two types of solutions in an appropriate ratio and then inducing a reaction for 30 to 60 minutes, and then performing the reaction again for 25 to 40 minutes in an incubator at 28 to 35 °C.

Next, as the step (2) according to the present invention, the step of preparing a cell culture coating composition is performed by mixing the prepared active solution and the cell culture fusion protein.

In this case, the fusion protein for cell culture and the active solution may be mixed by adjusting the content so that 1 to 100 parts by weight of the carbodiimide-based coupling agent is contained with respect to 100 parts by weight of the fusion protein for cell culture. If the amount of the carbodiimide-based coupling agent is less than 1 part by weight, cell adhesion may not occur or differentiation may occur, and if it exceeds 100 parts by weight, the cells may be detached after attachment, so it may be difficult to stably culture the cells.

In addition, the prepared active solution and the fusion protein for cell culture can be mixed, and then a reaction is induced for more than 0 to 2 hours to prepare the final cell culture coating composition.

Next, as the step (3) according to the present invention, the cell culture coating composition is treated on the surface of the non-porous substrate to form a cell culture coating layer.

The method of treating the prepared cell culture coating composition on the surface of the substrate may be a commonly used coating method. For example, if the substrate is a well plate, the composition may be dispensed using a pipette aid. After the cell culture coating composition is treated on the surface, a reaction can be induced in an incubator at 4 to 60 °C for more than 0 minutes to 2 hours to form the cell culture coating layer.

Thereafter, a washing process may be further performed, and for example, the washing process may be repeated 2 to 5 times in total for more than 0 to 30 minutes through tertiary distilled water. After the washing process, it can be naturally dried in the air, and through this, the cell culture substrate can be manufactured.

Table 1 below shows the amino acid sequences for the above-described mussel adhesive protein and functional peptide.

**[Table 1]**

| SEQ ID NO | Amino acid sequence |
|---|---|
| 1 | Ala Lys Pro Ser Tyr Pro Pro Thr Tyr Lys |
| 2 | Ala Lys Pro Ser Tyr Pro Pro Thr Tyr Lys Ala Lys Pro Ser Tyr Pro Pro Thr Tyr Lys |
| 3 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 4 | Glu Val His Ala Cys Lys Pro Asn Pro Cys Lys Asn Asn Gly Arg Cys Tyr Pro |
| | Asp Gly Lys Thr Gly Tyr Lys Cys Lys Cys Val Gly Gly Tyr Ser Gly Pro Thr Cys Ala Cys |
| 5 | Ala Asp Tyr Tyr Gly Pro Lys Tyr Gly Pro Pro Arg Arg Tyr Gly Gly Gly Asn Tyr Asn Arg Tyr Gly Gly Ser Arg Arg Tyr Gly Gly Tyr Lys Gly Trp Asn Asn Gly Trp Lys Arg Gly Arg Trp Gly Arg Lys Tyr Tyr Glu Phe Glu Phe |
| 6 | Ala Asp Tyr Tyr Gly Pro Lys Tyr Gly Pro Pro Arg Arg Tyr Gly Gly Gly Asn Tyr Asn Arg Tyr Gly Arg Arg Tyr Gly Gly Tyr Lys Gly Trp Asn Asn Gly Trp Lys Arg Gly Arg Trp Gly Arg Lys Tyr Tyr |
| 7 | Gly His Val His Arg His Arg Val Leu His Lys His Val His Asn His Arg Val Leu His Lys His Leu His Lys His Gln Val Leu His Gly His Val His Arg His Gln Val Leu His Lys His Val His Asn His Arg Val Leu His Lys His Leu His Lys His Gln Val Leu His |
| 8 | Ser Ser Glu Glu Tyr Lys Gly Gly Tyr Tyr Pro Gly Asn Ala Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Ser Ser |
| 9 | Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Gly Ser Ser |
| 10 | Tyr Asp Asp Tyr Ser Asp Gly Tyr Tyr Pro Gly Ser Ala Tyr Asn Tyr Pro Ser Gly Ser His Trp His Gly His Gly Tyr Lys Gly Lys Tyr Tyr Gly Lys Gly Lys |
| | Lys Tyr Tyr Tyr Lys Phe Lys Arg Thr Gly Lys Tyr Lys Tyr Leu Lys Lys Ala Arg Lys Tyr His Arg Lys Gly Tyr Lys Lys His Tyr Gly Gly Ser Ser Ser |
| 11 | Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Gly Ser Ser |
| 12 | Lys Tyr Tyr Tyr Lys Phe Lys Arg Thr Gly Lys Tyr Lys Tyr Leu Lys Lys Ala Arg Lys Tyr His Arg Lys Gly Tyr Lys Lys His Tyr Gly Gly Ser Ser Ser Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Gly Ser Ser Gly Gly Gly Asn Tyr Arg Gly Tyr Cys Ser Asn Lys Gly Cys Arg Ser Gly Tyr Ile Phe Tyr Asp Asn Arg Gly Phe Cys Lys Tyr Gly Ser Ser Ser Tyr Lys Tyr Asp Cys Gly Asn Tyr Ala Gly Cys Cys Leu Pro Arg Asn Pro Tyr Gly Arg Val Lys Tyr Tyr Cys Thr Lys Lys Tyr Ser Cys Pro Asp Asp Phe Tyr Tyr Tyr Asn Asn Lys Gly Tyr Tyr Tyr Tyr Asn Asp Lys Asp Tyr Phe Asn Cys Gly Ser Tyr Asn Gly Cys Cys Leu Arg Ser Gly Tyr Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ser Ser Glu Glu Tyr Lys Gly Gly Tyr Tyr Pro Gly Asn Ala Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Tyr Tyr Gly Gly Ser Ser Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 13 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ser Ser Glu Glu Tyr Lys Gly Gly Tyr Tyr Pro Gly Asn Ala Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Tyr Tyr Gly Gly Ser Ser Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 14 | Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ser Ser Glu Glu Tyr Lys Gly Gly Tyr Tyr Pro Gly Asn Thr Tyr His Tyr His Ser Gly Gly Ser Tyr His Gly Ser Gly Tyr His Gly Gly Tyr Lys Gly Lys Tyr Tyr Gly Lys Ala Lys Lys Tyr Tyr Tyr Lys Tyr Lys Asn Ser Gly Lys Tyr Lys Tyr Leu Lys Lys Ala Arg Lys Tyr His Arg Lys Gly Tyr Lys Lys Tyr Tyr Gly Gly Gly Ser Ser Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys Ala Lys Pro Ser Tyr Pro Pro Thr Tyr Lys |
| 15 | Lys Gly Gly Pro Gln Val Thr Arg Gly Asp Val Phe Thr Met Pro |
| 16 | Gly Ala Cys Arg Gly Asp Cys Leu Gly Ala |
| 17 | Lys Gly Gly Pro Gln Cys Val Thr Arg Gly Asp Val Phe Cys Thr Pro |
| 18 | Arg Gly Asp |
| 19 | Pro His Ser Arg Asn Ser Gly Ser Gly Ser Gly Ser Gly Ser Gly Arg Gly Asp Ser Pro |

### [Examples]

The present invention will be described in more detail through the following examples, but the following examples are not intended to limit the scope of the present invention, which should be construed to aid understanding of the present invention.

### <Example 1>

A 6-well plate made of sterilized polystyrene was prepared as a substrate. In this case, the 6-well plate was prepared without plasma treatment. Thereafter, 2 ml of the cell culture coating composition prepared in the following Preparation Example was dispensed in each well using a pipette aid, and then reacted in a constant temperature incubator to form a cell culture coating layer on the surface of the substrate. After washing three times for 10 minutes each using tertiary distilled water, the cell culture substrate was manufactured by drying in the air with the plate lid open in a clean bench.

### ^{∗} Preparation Example - Preparation of cell culture coating composition

The fusion protein for cell culture was prepared by binding the amino terminus of the functional peptide of SEQ ID NO: 15 to the carboxy terminus of the mussel adhesive protein of SEQ ID NO: 13. In this case, the fusion protein was prepared by a recombinant protein production method using E. coli.

Meanwhile, NaOAc, NaHCO₃, and 2-(N-morpholino)ethanesulfonic acid solutions dissolved in tertiary distilled water were first prepared to prepare the active solution, and then added in microtubes in which EDC and Sulfo-NHS reagents were respectively dispensed to prepare the EDC solution and Sulfo-NHS.

To prepare the cell culture coating composition, after the EDC solution was put into a conical tube, a Sulfo-NHS solution was added, and the fusion protein for cell culture was added to the prepared active solution while stirring, followed by stirring to prepare the cell culture coating composition. In this case, the cell culture coating composition contained 1 part by weight of EDC with respect to 100 parts by weight of the fusion protein for cell culture, and EDC and Sulfo-NHS were mixed in a weight ratio of 1:2, and the NaOAc contained in the coating composition was contained so as to be 100 parts by weight based on 100 parts by weight of EDC. In this case, the concentration of the fusion protein for cell culture in the cell culture coating composition was 0.05 mg/ml.

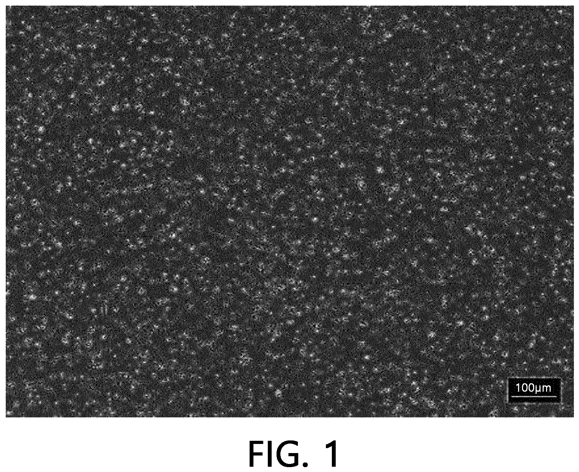

The SEM photograph of the surface of the cell culture coating layer according to Example 1 is as shown in FIG. 1, and it can be confirmed that the cell culture coating layer is formed by the aggregation of particles.

### <Example 2>

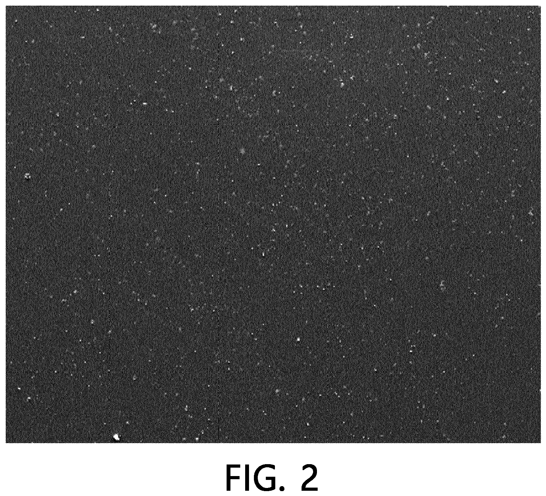

The cell culture substrate was manufactured in the same manner as in Example 1, except that the fusion protein for cell culture was changed to the fusion protein formed by binding the amino terminus of the functional peptide of SEQ ID NO: 19 to the carboxy terminus of the mussel adhesive protein of SEQ ID NO: 14. The SEM photograph of the surface of the cell culture coating layer according to Example 2 is as shown in FIG. 2, and it can be confirmed that the cell culture coating layer is formed by the aggregation of particles.

### <Comparative Example 1>

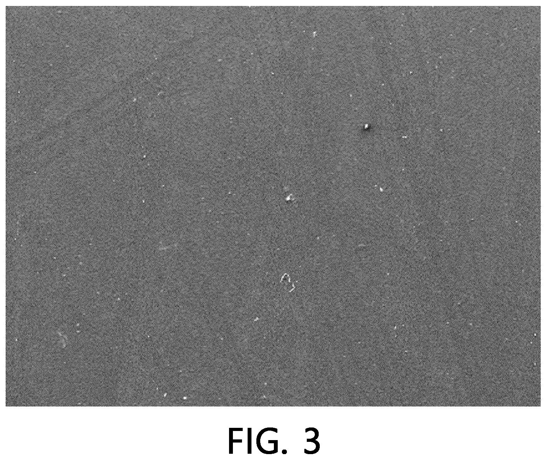

The cell culture substrate was manufactured in the same manner as in Example 1, but the 6-well plate that was not coated with the cell culture coating composition, and not subjected to plasma treatment, was used as the cell culture substrate. The SEM photograph of the surface of the cell culture coating layer according to Comparative Example 1 is as shown in FIG. 3, and it can be confirmed that it has a smooth surface.

### <Example 3>

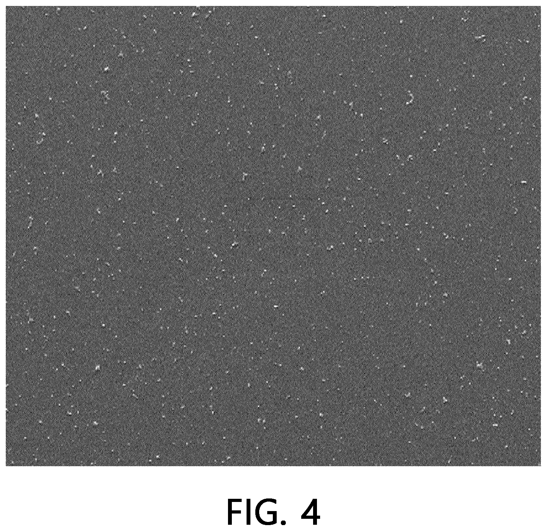

The cell culture substrate was manufactured in the same manner as in Example 2, but the fusion protein for cell culture was changed to the fusion protein formed by binding the amino terminus of the functional peptide of SEQ ID NO: 19 to the carboxy terminus of the mussel adhesive protein of SEQ ID NO: 14, and the material of the cell culture substrate was changed to polycarbonate. The SEM photograph of the surface of the cell culture coating layer according to Example 3 is as shown in FIG. 4, and it can be confirmed that the cell culture coating layer is formed by the aggregation of particles even when the material of the substrate to be coated is changed.

### <Examples 4 to 6>

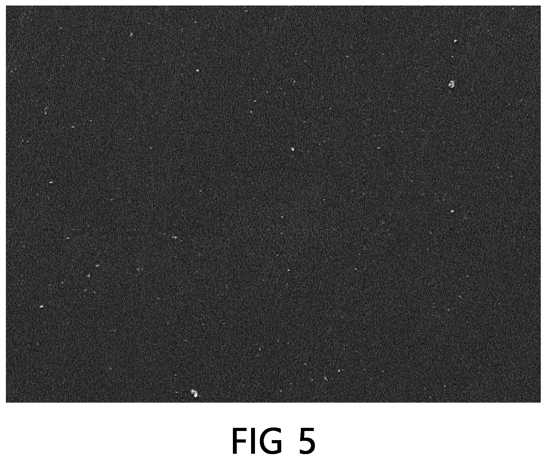

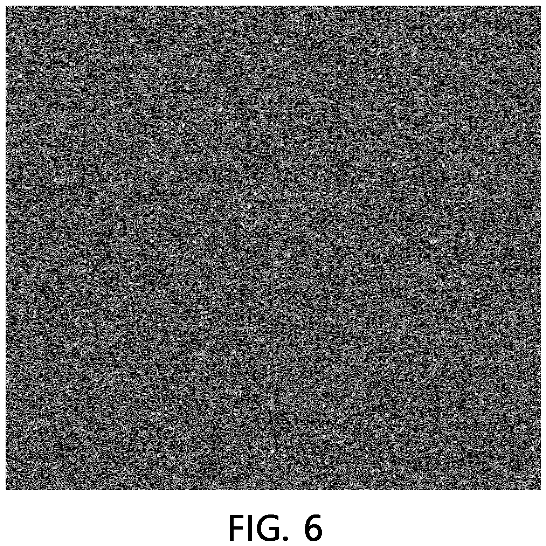

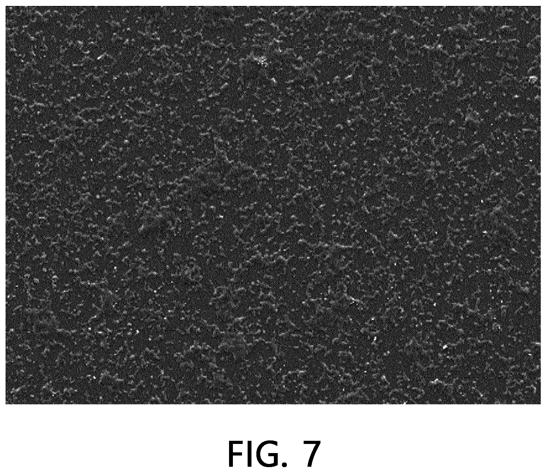

The cell culture substrate was manufactured in the same manner as in Example 1, except that the concentration of the fusion protein for cell culture in the cell culture coating composition was changed to 0.01 mg/ml, 0.1 mg/ml, and 0.5 mg/ml, respectively. The SEM photographs of the surfaces of the cell culture coating layers according to Examples 4 to 6 are as shown in FIGS. 5, 6 and 7, respectively. It can be confirmed that as the concentration of the fusion protein for cell culture increases, the particle diameter of the formed granules becomes larger and the number of particles decreases, and that large granules with irregular shapes are formed according to the bonding between the particles.

### <Comparative Example 2>

The cell culture substrate was manufactured in the same manner as in Example 1, except that the functional peptide of SEQ ID NO: 15 was used at the same concentration instead of the cell fusion protein.

### <Experimental Example 1>

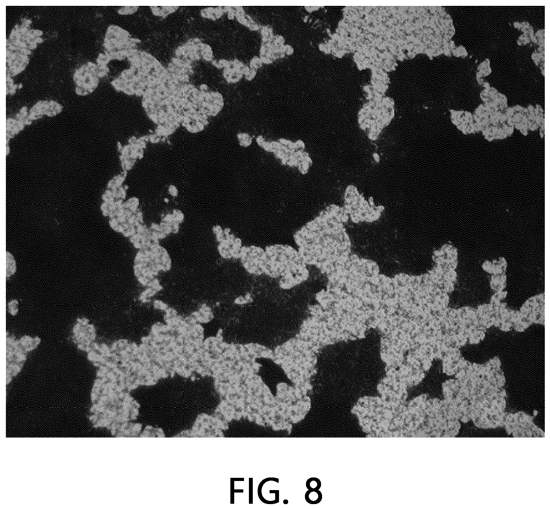

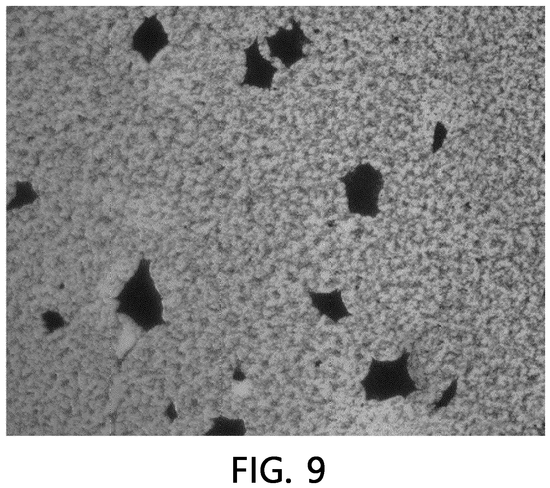

After dispensing the same amount of induced pluripotent stem cells to the cell culture substrates according to Example 1 and Comparative Example 2, the stem cells were cultured for 5 days using a stem cell culture medium (StemMACS^{™}). Then, the results of the cell culture were observed using a cell staining method, and the resulting photographs are shown in FIGS. 8 and 9.

As can be seen from FIG. 8, it can be seen that the cell culture substrate according to Example 1 exhibited excellent cell adhesion and growth, but the cell adhesion and growth were not properly performed in the cell culture substrate according to Comparative Example 2 of FIG. 9.

### <Experimental Example 2>

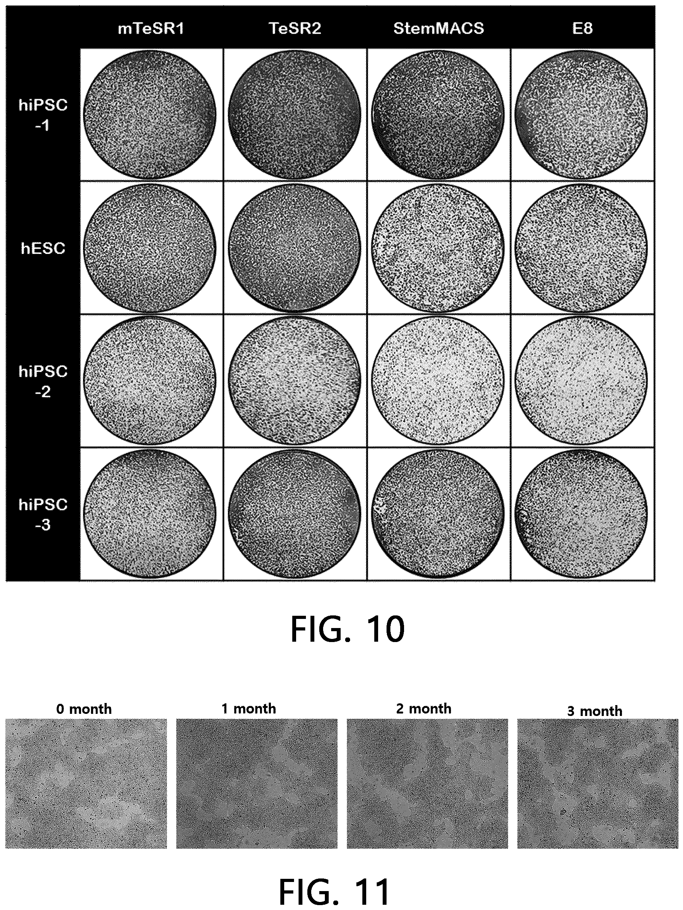

hiPSC-1, hESO, hiPSC-2, and hiPSC-3 were dispensed in each medium on the cell culture substrate according to Example 1, followed by culturing for 5 days using mTeSRl^{™}, TeSR2^{™}, StemMACS^{™}, E8^{™} mediums. Then, the result of the cell culture was observed with cell staining, and the resulting photograph was shown in FIG. 10.

As can be seen from FIG. 10, it can be seen that the cell culture substrate of Example 1 was suitable for various types of cell cultures and exhibited excellent compatibility with various types of mediums.

### <Comparative Examples 3 and 4>

A cell culture substrate was manufactured by coating Matrigel and Vitronectin-XF^{™}, which are commercially available as a cell culture coating composition, on a 6-well plate made of sterilized polystyrene as a substrate according to the coating composition manufacturer's protocol.

### <Experimental Example 3>

The cell culture substrates according to Example 1, Comparative Example 3 and Comparative Example 4 were subjected to an accelerated aging test according to the guidelines for setting of shelf-life evaluation of medical device and stability evaluation in the following manner, and then induced pluripotent stem cells were cultured and the storage stability was evaluated.

Specifically, in order to reproduce the real-time aging of the cell culture substrate within a shortened time, the cell culture substrate was stored at an elevated temperature (60 °C) for 0 month, 1 month, 2 months, and 3 months, and the aging period of each cell culture substrate was set to be 0 year, 1 year, 2 years, and 3 years.

After dispensing the same amount of induced pluripotent stem cells in each of the four cell culture substrates prepared for each Example and Comparative Example, the stem cells were cultured for 5 days using a stem cell culture medium (StemMACS^{™}), and photographed under an optical microscope to observe the result of cell culture. The resulting photographs are shown in FIGS. 11 (Example 1), 12 (Comparative Example 3) and 13 (Comparative Example 4), and the number of cultured cells is counted and shown in Table 2 below.

**[Table 2]**

| | Number of cultured cells per accelerated specimen (×10⁶) | | | |
|---|---|---|---|---|
| | 0 month | 1 month | 2 months | 3 months |
| Example 1 | 6.9 | 7.96 | 5.63 | 8.36 |
| Comparative Example 3 | 6.45 | 0 | 0 | 0 |
| Comparative Example 4 | 1.11 | 2.14 | 2.63 | 1.28 |

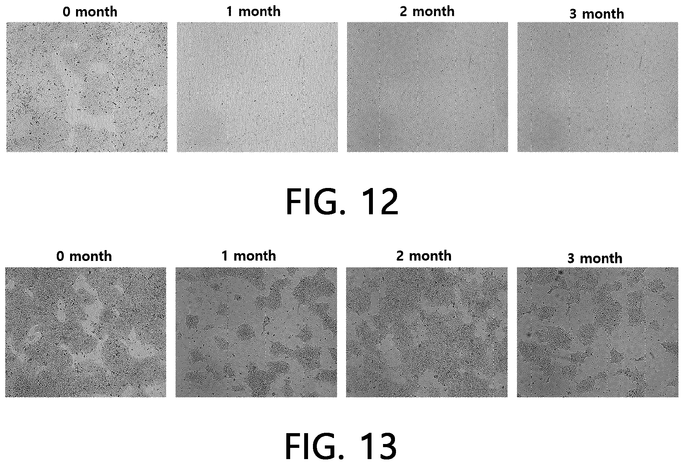

As can be seen from FIGS. 11 to 13 and Table 2, even when the cell culture substrates according to Example 1 had accelerated aging so that the aging period is 0, 1, 2, or 3 years, the cells were cultured in the cell culture substrates and the cell culture ability was high. However, in the cell culture substrate of Comparative Example 3, the cell culture ability was high when the aging period was 0 year, but cells were not cultured in the specimen in which the aging period was accelerated to 1 to 3 years. In addition, in the cell culture substrate of Comparative Example 4, cells were cultured in the 0 to 3 year accelerated specimens, but the number of cultured cells was significantly smaller compared to the specimen according to Example 1. Through this, it can be seen that the storage stability and cell culture ability of the cell culture substrate according to Example 1 are excellent.

Although one embodiment of the present invention has been described above, the spirit of the present invention is not limited to the embodiments presented herein. Those skilled in the art who understand the spirit of the present invention will be able to easily suggest other embodiments by including, changing, deleting, or adding components within the scope of the same spirit, but this is also said to be within the scope of the present invention.

## Claims

1. A cell culture substrate, comprising:
a substrate having a non-porous surface; and
a cell culture coating layer covering at least a portion of the non-porous surface,
wherein the cell culture coating layer is an aggregation of particles formed of a fusion protein for cell culture in which a functional peptide is bound to a mussel adhesive protein.

2. The cell culture substrate according to claim 1, wherein the functional peptide has a function of promoting any one or more of adhesion, migration, proliferation and differentiation of a cell.

3. The cell culture substrate according to claim 1, wherein the substrate is formed of any one or more materials selected from the group consisting of polycarbonate, polystyrene, polyimide, polyester, polyurethane, and glass.

4. The cell culture substrate according to claim 1, wherein the mussel adhesive protein is any one protein selected from the group consisting of amino acid sequences of SEQ ID NO: 1 to SEQ ID NO: 14, or a protein to which one or more amino acid sequences selected from the group are linked.

5. The cell culture substrate according to claim 1, wherein the functional peptide comprises an RGD sequence.

6. The cell culture substrate according to claim 1, wherein the functional peptide is any one or more peptides selected from the group consisting of amino acid sequences of SEQ ID NO: 15 to SEQ ID NO: 18, or a peptide to which one or more amino acid sequences selected from the group are linked.

7. A method for manufacturing a cell culture substrate, comprising the steps of:
(1) preparing an active solution containing a carbodiimide-based coupling agent and a reactive agent and a fusion protein for cell culture in which a functional peptide is bound to a mussel adhesive protein;
(2) preparing a cell culture coating composition by mixing the prepared active solution and the prepared fusion protein for cell culture; and
(3) forming a cell culture coating layer by treating the cell culture coating composition on a surface of a non-porous substrate.

8. The method for manufacturing a cell culture substrate according to claim 7, wherein the carbodiimide-based coupling agent is 1-ethyl-3-(3-dimethylaminopropyl carbodiimide hydrochloride (EDC) or N,N'-dicyclohexylcarboimide (DCC), and the reactive agent is N-hydroxysulfosuccinimide (Sulfo-NHS).

9. The method for manufacturing a cell culture substrate according to claim 7, wherein the carbodiimide-based coupling agent and the reactive agent are contained in the active solution in a weight ratio of 1: 0.1 to 10,
in the cell culture coating composition, 1 to 100 parts by weight of the carbodiimide-based coupling agent is mixed with respect to 100 parts by weight of the fusion protein for cell culture.

10. A cell culture coating composition for a non-porous cell culture substrate which forms a cell culture coating layer on a surface of the non-porous cell culture substrate, the cell culture coating composition comprising a fusion protein for cell culture in which a functional peptide is bound to a mussel adhesive protein, a carbodiimide-based coupling agent, and a reactive agent.
